**Europäisches Patentamt**

**European Patent Office**

**Office européen des brevets**

(11) Numéro de publication: **0 348 399 B1**

# FASCICULE DE BREVET EUROPEEN

(45) Date de publication de fascicule du brevet: **25.11.92**

(51) Int. Cl.5: **C07K 13/00**, C07K 7/06, C07K 7/10, C07K 3/28, A61K 39/395, C12N 15/00, C12Q 1/68

(21) Numéro de dépôt: **88901056.7**

(22) Date de dépôt: **08.01.88**

(86) Numéro de dépôt internationale : **PCT/FR88/00016**

(87) Numéro de publication internationale : **WO 89/06241 (13.07.89 89/15)**

(54) **SORBINE ET PEPTIDES DERIVES, ELEVANT L'ABSORPTION PAR LES MUQUEUSES.**

(43) Date de publication de la demande:
**03.01.90 Bulletin 90/01**

(45) Mention de la délivrance du brevet:
**25.11.92 Bulletin 92/48**

(84) Etats contractants désignés:
**AT BE CH DE FR GB IT LI LU NL SE**

(56) Documents cités:
**FR-A- 2 601 020**

**Chemical Abstracts, vol. 95, No. 7, 17 August 1981, (Columbus, Ohio, US), D. Pansu et al.: "Sorbin, a peptide contained in porcine upper small intestine which induces the absorption of water and sodium in the rat duodenum", see page 310, abstract 57581f, & Scand. J. Gastroenterol. 1981, 16(2), 193-9**

(73) Titulaire: **INSTITUT NATIONAL DE LA SANTE ET DE LA RECHERCHE MEDICALE (INSERM) 101, rue de Tolbiac F-75654 Paris Cédex 13(FR)**

(72) Inventeur: **Vagne, Monique 5, chemin de la Bussière F-69450 St-Cyr-au-Mont-d'Or(FR)**
Inventeur: **Pansu, Danielle Messia-sur-Sorne F-39570 Lons-le-Saunier(FR)**
Inventeur: **MUTT, Viktor Institut Karolinska S-104 01 Stockholm 60(SE)**
Inventeur: **JORNVALL, Hans Institut Karolinska S-104 01 Stockholm(SE)**

(74) Mandataire: **Lachassagne, Jacques Boite Postale 07 F-78430 Louveciennes(FR)**

Rank Xerox (UK) Business Services

**Description**

L'invention concerne des polypeptides et des peptides dérivés capables notamment de provoquer un accroissement de l'absorption, en particulier de l'eau, des électrolytes et des nutriments, par les muqueuses.

Elle a plus particulièrement pour objet des polypeptides de pureté élevée et des peptides ayant des séquences d'acides aminés en commun avec ces polypeptides.

On a déjà décrit des fractions biologiques obtenues, en particulier, à partir de muqueuses intestinales de porc, dotées des propriétés ci-dessus. Il s'agit cependant de compositions dans lesquelles le ou les principes actifs sont mélangés à de nombreux autres constituants susceptibles d'induire des effets secondaires non désirés qui les rend peu appropriés pour des applications thérapeutiques. Des polypeptides extraits d'intestins de porc ont été rapportés, mais aucune indication n'a été fournie sur la technique complète d'obtention de tels produits, ni sur leur séquence.

La mise au point d'un procédé de purification de polypeptides extraits d'intestins de porc a permis aux inventeurs d'isoler un polypeptide correspondant à un seul pic en HPLC et doté des activités indiquées plus haut. Ce polypeptide sera désigné dans la suite de la description et dans les revendications par le terme sorbine. Des dérivés de ce polypeptide et des fragments peptidiques ont pu être alors développés, dotés d'activités biologiques de grand interêt, en particulier présentant celles de la sorbine.

L'invention a donc pour but de fournir des polypeptides définis, hautement purifiés, utilisables comme réactifs et comme principes actifs de médicaments, et leurs anticorps. Elle a également pour but de fournir des peptides dérivés, c'est-à-dire correspondant plus spécialement à une ou plusieurs séquences d'acidés aminés de ces polypeptides, dotés d'une activité biologique donnée.

L'invention vise également l'obtention de ces polypeptides et de leurs dérivés selon différentes voies, notamment par extraction, par génie génétique ou, en particulier pour les peptides, par voie chimique.

L'invention a également pour but de mettre à profit les propriétés biologiques de ces peptides et polypeptides dans des compositions pharmaceutiques.

Les polypeptides de l'invention sont caractérisés en ce qu'au moins une partie de la séquence nucléotidique qui les exprime est capable de s'hybrider avec au moins une partie du gène de la sorbine de séquence (I) suivante en acides aminés :

```
      1     5     10      15  20    25    30    35    40
  >MRAATPLQTVDRPKDTYKTMFKQIHMVHKPDDDTKMYNTP
        45    50  55    60    65    70    75    80
  YTYNAGLYNSPYSAQSHPAAKTQTYRPLSKSHSDNGTDAF
        85    90    95    100   105   110   115   120
  KDASSPVPPPHVPPPVPPLRPRDRSSTEKHDRDPPDRKVD
       125   130   135   140   145   150 153
  TRKFRSEPRSIFEYEPGKSSILQHERPVTKPQA- NH2
```

De tels polypeptides sont capables, avantageusement, de provoquer un accroissement de l'eau, des électrolytes et des nutriments par les muqueuses, en particulier les muqueuses digestives.

Pour illustrer cette séquence et celles données ci-après, on utilise la nomenclature suivante à une lettre des acides aminés:

Alanine = ala = A/    arginine = arg = R/    asparagine = asn = N/    acide    aspartique = asp = D/    acide glutamique = glu = E/    glutamine = gln = Q/    glycine = gly = G/    histidine = his = H/    isoleucine = ile = I/ leucine = leu = L/    lysine = lys = = K/    méthioniné = met = -M/    phénylalanine = phe = F/    proline = pro = P/ sérine = ser = S/ thréonine = thr = T/ trytophane = trp = W/ tyrosine = tyr = Y/ valine = val = V/.

D'une manière préférée, l'invention vise des polypeptides et peptides dérivés renfermant au moins une partie d'un heptapeptide de séquence (II) :

P.V.T.K.P.Q.A. - $NH_2$

Les polypeptides et peptides selon l'invention sont encore caractérisés en ce qu'ils renferment au moins une partie d'un décapeptide répondant à la séquence (III) en acides aminés :

H.E.R.P.V.T.K.P.Q.A - $NH_2$.

Selon une autre caractéristique, les polypeptides et peptides de l'invention comprennent au moins une partie de la séquence suivante (IV) de 40 acides aminés :

$$\begin{array}{cccccc} 1 & 5 & 10 & 15 & 20 & 25 \end{array}$$

P.P.D.R.K.V.D.T.R.K.F.R.S.E.P.R.S.I.F.E.Y.E.P.G.K.S.S.I.

$$\begin{array}{ccc} 30 & 35 & 40 \end{array}$$

L.Q.H.E.R.P.V.T.K.P.Q.A-NH$^2$

L'invention vise, en particulier, un polypeptide ou sorbine qui présente, en plus des caractéristiques qui précèdent, une pureté élevée. Ainsi, la sorbine est caractérisée par un seul pic en HPLC avec un temps de rétention de 20 à 22 minutes sur une colonne micro-Bondapak C18, Waters (3,9 x 300 mm), avec un débit de 1,5 ml/minute, en utilisant un solvant A constitué par du méthanol à 40 % et de l'acide trifluoroacétique (TFA) à 0,1 % et un solvant B formé de méthanol à 80 % et de TFA à 0,1 %, le gradient de B étant de 0 à 75 % en 30 minutes.

Dans la colonne de chromatographie ci-dessus, en utilisant le même système de solvant mais un gradient de 0 à 100 % en 20 minutes, la sorbine de l'invention correspond au pic obtenu avec un temps de rétention de 15 à 16 minutes.

La sorbine purifiée telle qu'obtenue par HPLC comme indiqué ci-dessus comporte plus spécialement 153 acides aminés et répond à la séquence (I) en acides aminés. Le poids moléculaire de cette chaine peptidique est de 17 483.

Divers fragments peptidiques peuvent être obtenus à partir des polypeptides ci-dessus, notamment de la sorbine, ces fragments ayant le même type d'activité que ces polypeptides à l'égard de l'absorption par les muqueuses intestinales ou des activités biologiques différentes.

Des peptides préférés comprennent au moins une partie de la séquence (IV).

D'autres peptides préférés comprennent ou correspondent aux hepta et décapeptides répondant aux séquences (II) et (III) d'acides aminés indiquées plus haut.

Il va de soi qu'il est possible de remplacer certains acides aminés des séquences I à IV sans modifier l'activité ou, inversement, en leur conférant, grâce à ces modifications, un effet opposé comme le montre l'un des exemples ci-après.

En particulier, les acides aminés basiques de ces séquences peuvent être remplacés par d'autres acides aminés basiques tels que l'arginine, la lysine et l'histidine. De même les acides aminés à groupements acides tels que l'acide aspartique et l'acide glutamique peuvent être échangés.

Il est également possible d'introduire des groupes de substitutions sur les acides aminés de la chaîne peptidique pour retarder son métabolisme et améliorer sa durée d'action.

L'invention concerne également les anticorps capables de reconnaître spécifiquement les polypeptides et peptides définis ci-dessus.

L'invention vise en outre les séquences d'ARN-m correspondant aux polypeptides et peptides dérivés définis ci-dessus. Elle vise en particulier la séquence d'ARN-m correspondant à la séquence (I) d'acides aminés.

Les fragments d'ADN capables de coder pour au moins une partie des polypeptides et de leurs dérivés définis ci-dessus entrent également dans le cadre de l'invention ainsi que les produits codés, en particulier les polypeptides et peptides ayant une activité sur la capacité d'absorption par les muqueuses.

Ces séquences nucléotidiques sont également caractérisées par leur capacité d'hybridation avec un gène capable d'exprimer au moins une partie de la sorbine de séquence (I).

L'invention vise également des moyens d'obtention de la sorbine et de ses dérivés selon l'invention.

Un procédé d'obtention comprend la purification par chromatographie liquide et par HPLC de peptides extraits d'organes de mammifères.

Selon des techniques classiques, ces peptides sont extraits par de l'acide acétique, relargués par du chlorure de sodium puis purifiés par précipitation alcoolique. Le précipité de peptides obtenu est soumis, conformément au procédé de l'invention, aux étapes suivantes :

- purification par chromatographie sur carboxyméthylcellulose (CMC) avec élution par du bicarbonate d'ammonium 0,04M,
- récupération de la fraction active et nouvelle chromatographie sur CMC, avec élution par du bicarbonate d'ammonium 0,03M,

- récupération de la fraction active soumise à au moins une purification par HPLC (high pressure liquid chromatography) avec un solvant A constitué par de l'eau avec 0,1% d'acide trifluoroacétique et un solvant B constitué par de l'acétonitrile contenant 0,1% de TFA.

Dans un mode de réalisation préféré, les purifications successives par HPLC sont réalisées comme suit:

- on utilise une colonne micro-Bondapak C 18 waters (7,8 x 300 mm) et un gradient du solvant B de 0 à 20% en 10 minutes puis de 20 à 40 en 40 minutes et un débit de 3 ml/minute, on élue le produit actif avec un temps de rétention de 37 à 39 minutes,
- on utilise une colonne micro-Bondapak CN Waters (3,9 x 300 mm), avec un débit de 1,5 ml/minute et un solvant A constitué par de l'eau et 0,1% de TFA et un solvant B comprenant de l'acétonitrile et 0,1% de TFA, on ajoute le solvant B en gradient à raison de 0 à 20% en 10 minutes, puis 20 à 40% en 40 minutes, on élue la fraction active avec un temps de rétention de 37 à 41 minutes,
- on utilise une colonne micro-Bondapak C18, Waters (3,9 x 300 mm) avec un débit de 1,5 ml/minute, un solvant A, formé par du méthanol 40% et du TFA 0,1%, un solvant B constitué par du méthanol 80% et du TFA 0,1%, le gradient de B étant de 0 à 75% en 30 minutes, le temps de rétention de la sorbine étant de 20 à 22 minutes.

Selon une autre voie, les polypeptides et leurs dérivés sont obtenus, conformément à l'invention, par la technique de l'ADN recombinant.

Ainsi, partant d'une séquence nucléotidique codant pour la sorbine (séquence obtenue soit directement à partir du génome de la sorbine, soit à partir de l'ARN-m correspondant, soit par synthèse), on procède à la fabrication de cette protéine en ayant recours à des techniques maintenant devenues classiques dans le domaine du génie génétique.

L'invention vise également les moyens intermédiaires tels que vecteurs et souches bactériennes utilisables pour l'obtention des polypeptides.

Selon encore une autre voie, les polypeptides et leurs dérivés, plus spécialement les fragments peptidiques de ces polypeptides, sont préparés par voie de synthèse.

On a recours à cet effet aux techniques classiques, en particulier en phase solide.

Une méthode de ce type particulièrement appropriée est constituée par la technique des FMOC aminoacides, développée par Carpino et al dans J.O.C 37, 3404 - 3409 (1972) et Meinhofer dans Int. J. Pept. Pro. Res; 1978, 11: 246-249.

Les FMOC aminoacides (fluorénylméthoxycarbonyl) sont utilisés soit sous forme d'anhydrides (instables donc préparés extemporanément par activation par le dicyclohexylcarbodiimide dans le dichlorométhane), soit sous forme d'esters actifs (esters de pentafluorophényle (PFP)) via la formation d'une liaison ester avec le DMAP (4-diméthylaminopyridine) dans le DMF (diméthylformamide).

Le couplage s'effectue par circulation en continu dans une colonne de résines pendant 25 à 60 minutes environ. On utilise avantageusement les résines du commerce constituées par des composites de Kieselguhr et de gel de polyamide polymerisé dans les pores de la matrice macroporeuse de Kieselguhr, telles que : la résine pepsyn KA (qui libère un peptide terminé par un amino-acide à fonction acide libre par traitement par le TFA 95%), la résine pepsyn B (qui conduit à un peptide dont le C terminal est amidé, après clivage par le méthanol ammoniaqué) et la résine pepsyn KH (qui conduit à un amino-acide terminal protégé après clivage par le TFA 1%). La déprotection du N terminal s'effectue avec 20% de pipéridine dans le DMF. Les acides aminés suivants sont ajoutés un par un en présence ou non de catalyseur dans le DMF (HOBT, 1 hydroxybenzotriazole) puis déprotégé avant l'addition du suivant. Entre chaque étape de couplage et de déprotection, l'excès des réactifs est éliminé par lavage avec le DMF.

En fin de synthèse, le peptide est décroché de la résine par le solvant approprié suivant le type de résine comme indiqué plus haut. Les protections des groupements latéraux sont éliminés par le TFA. Une purification par chromatographie liquide haute pression est ensuite réalisée pour éliminer des peptides incomplets ou des peptides incomplètement déprotégés et obtenir des peptides parfaitement caractérisés par leur temps de retention, leur composition en amino-acides et leur séquence.

Cette technique n'est pas exclusive et ne pourra avoir recours à d'autres techniques telles que celles décrites ci-dessous, en solution homogène ou en phase solide.

Par exemple, on aura recours à la technique de synthèse en solution homogène décrite par HOUBENWEYL dans l'ouvrage intitulé "Methode der organischen Chemie" (Méthode de la chimie organique) édité par E. Wènsch., vol. 15-I et II, THIEME, Stuttgart 1974.

Cette méthode de synthèse consiste à condenser successivement deux à deux les aminoacyles successifs dans l'ordre requis, ou à condenser des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments à l'exception des fonctions amine de l'un et carboxyle de l'autre ou vice versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-éthyl-3-(3-diméthyl-aminopropyl)-carbodiimide. Lorsque l'aminoacyle mis en oeuvre possède une fonction amine supplémentaire (cas de la lysine par exemple) ou une autre fonction acide (cas par exemple de l'acide glutamique), ces fonctions seront par exemple protégées, par des groupes carbobenzoxy ou t-butyloxycarbonyle, en ce qui concerne les fonctions amine, ou par des groupes t-butylester, en ce qui concerne les fonctions carboxyliques. Il en ira de même de la protection de toute autre fonction réactive. Par exemple, lorsque l'un des aminoacyles considérés contient une fonction SH (par exemple la cystéine), on pourra avoir recours à un groupe acétamidométhyle ou paraméthoxybenzyle.

Dans le cas de la synthèse progressive, acide aminé par acide aminé, la synthèse débute de préférence par la condensation de l'amino-acide-C-terminal avec l'aminoacide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R.D. MERRIFIELD dans l'article intitulé "Solid phase peptide synthesis" (J. Am. Chem. Soc., 45, 2149-2154).

Pour fabriquer une chaîne peptidique selon le procédé de MERRIFIELD, on a recours à une résine polymère très poreuse, sur laquelle on fixe le premier aminoacide C-terminal de la chaine. Cet aminoacide est fixé sur la résine par l'intermédiaire de son groupe carboxylique et sa fonction amine est protégée, par exemple par le groupe t-butyloxycarbonyle.

Lorsque le premier aminoacide C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième aminoacide qui fournit le second aminoacyle de la séquence recherchée, à partir du résidu aminoacyle C-terminal sur la fonction amine déprotégée du premier aminoacide C-terminal fixé sur la chaîne. De préférence, la fonction carboxyle de ce deuxième aminoacide est activée, par exemple par le dicyclohexylcarbodiimide, et la fonction amine est protégée, par exemple par le t-butyloxycarbonyle.

On obtient ainsi la première partie de la chaîne peptidique recherchée, qui comporte deux amino-acides, et dont la fonction amine terminale est protégée. Comme précédemment, on déprotège la fonction amine et on peut ensuite procéder à la fixation du troisième aminoacyle, dans les conditions analogues à celles de l'addition du deuxième aminoacide C-terminal.

On fixe ainsi, les uns après les autres, les acides aminés, qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents aminoacides constituant la chaine peptidique et on détache le peptide de la résine par exemple à l'aide d'acide fluorhydrique.

L'étude des propriétés pharmacologiques des polypeptides et de leurs dérivés a mis en évidence leur effet avantageux sur l'absorption notamment de l'eau, des électrolytes et des nutriments par les muqueuses, en particulier les muqueuses digestives.

Les travaux effectués ont montré que la sorbine est répartie assez largement en quantités réduites mais avec une prédominence notable au niveau de l'hypophyse, des surrénales, des bronches et du duodénum.

La présence de la sorbine dans des tissus divers attribue à ce peptide et à ses fragments, un rôle ubiquitaire dans le transport cellulaire des électrolytes, et en particulier du chlore à tous les niveaux et en particulier du tractus digestif, des sécrétions bronchiques et du système nerveux central. Au niveau du système nerveux central, il intervient dans les troubles du comportement liés en particulier à un déséquilibre ionique.

Ces propriétés avantageuses s'accompagnent d'une grande innocuité.

L'invention vise donc des compositions pharmaceutiques renfermant dans leur principe actif une quantité efficace des polypeptides et de leurs dérivés tels que définis ci-dessus, en association avec un véhicule pharmaceutique.

Les compositions pharmaceutiques sont administrables par voie intraveineuse ou par voie buccale.

Pour l'administration par voie intraveineuse, on a recours à des ampoules injectables. Ces préparations renferment avantageusement de 10 à 50 picomoles par unité de prise, de préférence de 20 à 40 picomoles.

D'autres formes d'administration sont constituées par des comprimés, tablettes, gélules, à libération intestinale retardée et renferment de 50 à 500 picomoles de principe actif, de préférence de 150 à 250 picomoles.

Compte tenu de leurs propriétés sur l'absorption, ces compositions sont utilisables notamment pour

- le traitement des diarrhées infectieuses et des toxicoses aigues du nourrison par l'effet bénéfique d'une augmentation de l'absorption de l'eau et des électrolytes,
- le traitement adjuvant de la réanimation parentérale lors de la réinduction de l'alimentation entérale, au décours de maladies infectieuses, d'interventions chirurgicales, en particulier sur la sphère digestive,
- le traitement de certaines malabsorptions chroniques par augmentation de l'absorption des glucides et des aminoacides liée à l'augmentation de l'absorption de l'eau et des électrolyses,
- le traitement de certains troubles électrolytiques et notamment ceux de la mucoviscidose, caractérisés par un trouble de la réabsorption des électrolytes au niveau des glandes sudoripares, salivaires, bronchiques, de l'intestin et du pancréas,
- le traitement des obésités et des surcharges hydriques par les dérivés substitués des polypeptides et peptides de l'invention qui suppriment l'absorption de l'eau, des électrolytes et des nutriments.

L'invention concerne encore les réactifs biologiques dont les principes actifs sont constitués par la sorbine et ses dérivés. Ces réactifs biologiques peuvent être utilisés comme références ou étalons dans des études d'action éventuelles de composés sur l'absorption de l'eau et des électrolytes.

D'autres caractéristiques et avantages de l'invention apparaîtront dans les exemples qui suivent relatifs à la préparation de sorbine pure et de fragments peptidiques et à leur activité biologique.

Exemple 1 : Préparation de sorbine pure par extraction à partir d'organes de mammifères.

Au cours d'une suite d'opérations 1) on isole tout d'abord, selon des techniques classiques, un précipité de peptides sous forme de sels, par extraction à partir d'intestins de porc.

le gâteau de sel obtenu est soumis ensuite à un processus de purification 2) pour isoler la sorbine.

1: extraction de peptides à partir d'intestins de porc selon la technique décrite par V. MUTT, Clin. Endocri. 1976, 5, supp. 175S-183S.

Des intestins de porc correspondant au 1/3 supérieur de l'intestin, sont prélevés à l'abattoir et portés à l'ébullition dans de l'eau puis conservés congelés. Ils sont ensuite coupés en morceaux et ils sont traités par macération dans de l'acide acétique 0,5 M pendant 12 heures. Le macérat est filtré, adsorbé sur de l'acide alginique, élué par de l'acide chlorhydrique (HCl)0,2 M. Les peptides sont précipités de l'éluat par du chlorure de sodium à saturation (1 tonne d'intestins donne 1 kg de précipité).

Le précipité est dissous dans de l'eau à raison de 20 g pour 100 ml. Deux volumes d'éthanol à 95% sont ajoutés. Le pH de la solution est ajusté à 7,2 avec de la soude (NaOH) par l'intermédiaire d'une électrode en verre et d'un pHmètre. Le précipité qui se forme est filtré et écarté. Un volume d'éthanol à 95%, à la température de -20°C, est alors ajouté au filtrat clarifié. Le mélange est stocké à -20° C pendant 48 heures. Le précipité est recueilli par filtration à -20° C. Le précipité est dissous dans du tampon phosphate 0,03 M à pH 6,4 et adsorbé sur de la carboxyméthylcellulose (CMC) et élué avec le même tampon renfermant du chlorure de sodium à une concentration de 0,3 M. Le produit est précipité de cette solution par du chlorure de sodium à saturation. Le rendement de cette étape est de 22,5 g pour 1 kg de l'extrait précédent.

Le produit est alors séparé en plusieurs fractions par chromatographie sur Séphadex G 25 en tampon acide acétique 0,2 M. Le produit est précipité par du chlorure de sodium à saturation. La quantité récupérée dans la fraction active est environ de 9 g.

2: étapes de purification: Le gâteau de sel obtenu à l'étape précédente est dissous dans de l'eau à la concentration de 10 g pour 100 ml. Deux volumes d'éthanol à 95% sont ajoutés et le pH est porté à 7,2 à l'aide d'une solution renfermant 1 volume de NaOH 1 M et 2 volumes d'éthanol 95%. Un volume d'éthanol à 95%, identique au nouveau volume total obtenu, amené à la température de -20° C, est ajouté au mélange qui est placé 48 heures à - 20° C. Le précipité est recueilli par filtration, puis séché par lavage par l'éthanol pur, puis l'éther et enfin séché sous vide pendant plusieurs heures. Son poids est de 5 g environ. Cette étape a permis de sécher le produit et de se débarrasser d'une partie du sel.

Le produit est alors soumis à une chromatographie sur carboxyméthylcellulose (CMC) après dissolution dans du bicarbonate d'ammonium 0,02 M. Il est absorbé puis élué par du bicarbonate d'ammonium 0,04 M. La fraction active ainsi éluée représente 335 mg.

La fraction obtenue est dissoute à nouveau dans du bicarbonate d'ammonium 0,03 M et soumise à une nouvelle chromatographie sur CMC et l'activité est repérée dans les fractions éluées entre 25 et 35 fois le volume total de la colonne. Le poids de la fraction active ne représente plus que 19,2 mg environ.

L'étape suivante est réalisée par chromatographie liquide haute pression (HPLC) en utilisant un solvant A constitué par de l'eau avec 0,1% d'acide trifluoracétique (TFA) et un solvant B constitué par de l'acétonitrile contenant 0,1% de TFA. Sur une colonne micro-Bondapak C18 Waters (7,8 x 300 mm), un gradient du solvant B de 0 à 20% en 10 minutes puis de 20 à 40 en 40 minutes et un débit de 3 ml/minute, le produit actif est élué avec un temps de rétention de 37 à 39 minutes.

L'étape suivante consiste en une HPLC, sur colonne micro-Bondapak CN Waters (3,9 x 300 mm) à un débit de 1,5 ml/minute, en utilisant un solvant A constitué par de l'eau et 0,1% de TFA et un solvant B comprenant de l'acétonitrile et 0,1% de TFA. Le solvant B est ajouté en gradient à raison de 0 à 20% en 10 minutes, puis de 20 à 40% en 40 minutes. La fraction active est éluée avec un temps de rétention de 37 à 41 minutes.

L'étape finale consiste en une HPLC, sur colonne microbondapak C18, Waters (3,9 x 300 mm) à un débit 1,5 ml/minute, en utilisantun solvant A : méthanol 40% + TFA 0,1% solvant B : méthanol 80% + TFA 0,1%. Le gradient de B est de 0 à 75% en 30 minutes. Le temps de rétention de la sorbine est alors de 20 à 22 minutes.

Le contrôle de pureté réalisé sur la même colonne avec le même système de solvant et un gradient de 0 à 100% en 20 minutes, montre qu'un seul pic est obtenu dont le temps de rétention est de 15 à 16 minutes.

On récupère 0,462 mg de sorbine à la fin de la purification portant sur une tonne d'intestins.

a - CARACTERISATION DE LA SORBINE

La pureté de la sorbine a été mise en évidence par l'obtention d'un seul pic en HPLC et une seule bande en électrophorèse sur gel d'acrylamide.

La composition en aminoacides a été déterminée après hydrolyse de la molécule par de l'acide chlorhydrique 6 N et 0,5 % de phénol à 106°C pendant 24 heures et le dosage sur un analyseur Beckman. Deux lots ont été analysés et les résultats ont été comparés à ceux obtenus par la séquence totale.

b - COMPOSITION EN AMINO-ACIDES DE LA SORBINE

La détermination a eu lieu sur 2 lots différents appelés I et II. La moyenne M̄ des deux lots est donnée. Cette composition est comparée à celle calculée d'après la séquence (Seq.) III donnée ci-dessus.

| | Lot | I | II | M | Seq |
|---|---|---|---|---|---|
| trp | | - | - | - | - |
| lys | | 13,2 | 13,3 | 13,3 | 13 |
| his | | 6,5 | 7,5 | 7 | 7 |
| arg | | 10,8 | 9,8 | 10,3 | 12 |
| asp | | | | 13 | |
| | | 18,4 | 14,7 | 16,6 | 17 |
| asn | | | | 4 | |
| thr | | 12,3 | 11 | 11,7 | 13 |
| ser | | 15 | 13,2 | 14,1 | 14 |
| glu | | | | 5 | |
| | | 12 | 12,3 | 12,2 | 11 |
| gln | | | | 6 | |
| pro | | 20,9 | 24,6 | 22,8 | 23 |
| gly | | 4 | 4,2 | 4,1 | 3 |
| ala | | 9,3 | 9,9 | 9,6 | 9 |
| val | | 6,9 | 7,1 | 7 | 7 |
| met | | 3,9 | 4,1 | 4 | 4 |
| ile | | 3 | 3 | 3 | 3 |
| leu | | 5,1 | 5,6 | 5,5 | 5 |
| tyr | | 7,8 | 8,6 | 8,2 | 8 |
| phe | | 4 | 4,4 | 4,1 | 4 |
| total | | | | 153 | |

La détermination du N terminal par la méthode de dansylation classique n'a pas permis de mettre en évidence de groupe terminal, ce qui indique que le groupe terminal est bloqué par un groupement soit N-acétyle, soit N-formyle ou par un groupement moins classiquement rencontré. Par contre, la présence de motifs epsilon-lysine et de motifs o-tyrosine dans la molécule indique que la réaction s'est déroulée convenablement puisque ces deux aminoacides prennent la dansylation à leur groupement $NH_2$ ou OH libres et non en position terminale.

c - DETERMINATION DE LA STRUCTURE DE LA SORBINE PURE:

La détermination de la structure de la sorbine a été réalisée à l'aide de séquenceurs Applied et Beckman sur la molécule préalablement scindée par 3 procédés différents, puisque la séquence directe est impossible du fait du bloquage du N terminal par un radical.

L'action du bromure de cyanogène a coupé la molécule au niveau des résidus méthionine (M ou MET). 5 fragments ont été séquencés.

La GLU-PROTEASE a permis de couper la molécule après les résidus Lysine ou Arginine: 30 fragments ont été séquencés.

Le chevauchement des différentes séquences obtenus pour chacun des fragments a permis de reconstituer la molécule entière de sorbine.

Exemple 2 : Procédé d'obtention d'un fragment peptidique de 40 acides aminés selon l'invention.

Le fragment peptidique contenant les 40 derniers amino-acides et qui renferme l'activité de la molécule entière sur l'absorption d'eau et des électrolytes a pu être obtenu par clivage non spécifique par l'acide formique 70% après 72 heures de séjour à la température du laboratoire et être produit de façon non spécifique par clivage par le bromure de cyanogène. La coupure par le bromure de cyanogène est réalisée comme suit: on utilise un flacon en forme de poire de 100 ml, muni d'un rodage s'adaptant sur un rotavapor (ballon muni d'un réfrigérant sur lequel on applique le vide). Quand l'échantillon a été réparti sur toute la paroi, puis séché complètement, on ouvre le système et on ajoute 700 ul d'acide formique, 300 ul d'eau et 0,2 g de bromure de cyanogène. On agite et on laisse en contact pendant 24 heures à la température ordinaire. Le lendemain, l'échantillon est séché comme auparavant à l'aide du rotavapor. Lorsque l'échantillon est sec on reprend par 0,5 ml d'acide formique, 0,5 ml d'eau et on sèche de nouveau. Les produits coupés par le bromure de cyanogène sont ensuite séparés par HPLC sur une colonne micro Bondapk C18 waters avec un gradient comprenant les solvants A et B rapportés ci-dessus.

Avec un gradient du solvant B de 0 à 40% en 40 minutes, le peptide actif a été séparé au temps de rétention de 38 à 40 minutes, et comprend suivant les teneurs indiquées les acides aminés ci-après :

- lysine 0,61 nanomoles,
- histidine 0,18,
- arginine 0,72,
- acide aspartique 0,36,
- sérine 0,72,
- acide glutamique 0,92,
- proline 0,95,
- valine 0,32,
- isoleucine 0,18,
- leucine 0,20,
- tyrosine 0,25,
- phénylalanine 0,29.

Exemple 3 : Procédé d'obtention de la sorbine par génie génétique.

La séquence nucléotidique codant pour la sorbine est insérée en phase au milieu de la séquence nucléotidique (et en tout cas derrière l'ATG d'initiation) d'un gène cloné avec son promoteur (ou auquel on adjoint en amont, un promoteur étranger). L'utilisation d'adaptateurs ("linkers"), permet d'envisager l'utilisation de plusieurs types de vecteurs d'expression différents selon le promoteur considéré : par exemple

a) des promoteurs bactériens

C'est notamment le cas des plasmides contenant la région promoteur-opérateur de l'opéron lactose d'E. Coli (opéron lac), suivie de la partie 5' du gène de la $\beta$-galactosidase. Ces vecteurs, du type pPC (CHARNAY et al, Nucleic Acid Research 1978, tome V, pp. 4479-4494) permettent l'insertion du fragment codant pour la sorbine au site EcoRI situé à 21 nucléotides derrière l'ATG d'initiation de la $\beta$-galactosidase. La protéine exprimée comporte alors pour l'extrémité N-terminale les sept (ou huit) premiers acides aminés de la $\beta$-galactosidase bactérienne, suivis des acides aminés de la sorbine.

b) des promoteurs de phage

Il s'agit notamment de plasmides contenant la région promoteur-opérateur de l'opéron de gauche (P$_L$) ou de l'opéron de droite (P$_R$) du phage λ. Ces vecteurs, respectivement du type pKC30 (ROSENBERG, Nature 1981, Vol. 292, p. 128) ou pRL447 (ZABEAU ; dérivé de pRC5 et de pLG400, ce dernier étant décrit dans Cell, 1980, Vol. 20, pp. 543-553) permettant d'effectuer l'insertion du fragment codant pour la sorbine au sein des séquences nucléotidiques codant respectivement pour l'extrémité N terminale du produit du gène N ou pour celle du produit du gène cro.

Ces systèmes de vecteurs sont propagés à 30°C dans des bactéries lysogénisées par un phage λ à répresseur thermosensible (cI 857) ou en présence de plasmides porteurs du même gène (cI 857) codant pour un répresseur thermosensible. Ils demeurent inactifs, du fait du répresseur, tant que la culture est maintenue à 30°C. Le transfert de la culture à 42°C est suivi de l'activation des promoteurs de λ(P$_L$ ou P$_R$) portés par le plasmide recombinant, du fait de l'inactivation du répresseur du gène cI 857.

c) des promoteurs viraux

On utilise par exemple le virus SV40 comme vecteur. Dans ce cas, on utilise le promoteur viral tardif et le fragment codant pour la sorbine est inséré à la place de tout ou partie de la région codant pour les protéines tardives de SV40. On construit ainsi des ADN de SV40 substitués dans lesquels les séquences codant pour les protéines de capside de ce virus sont remplacées par la séquence codant pour la sorbine dans des conditions plaçant cette séquence sous le contrôle desdits promoteurs.

Les vecteurs recombinants obtenus peuvent alors être utilisés pour la transformation de cellules de mammifères, par exemple des cellules CHO, dont les polymérases reconnaissent les promoteurs de SV40, et ce dans des conditions permettant l'expression de la séquence codant pour la sorbine. Celle-ci peut ensuite être isolée à partir d'extraits de cellules transformées, par exemple par mise en contact d'une solution de ces extraits avec des anticorps anti-sorbine immobilisés sur un support insoluble et par récupération de la sorbine à partir du complexe sorbine-antisorbine retenu sur le support.

d) des promoteurs de virus animaux portés par des plasmides bactériens

Des plasmides utilisables portent les promoteurs du gène de la thymidine-kinase du virus de l'herpès (pAGO), du gène de l'antigène HBS du virus de l'hépatite B (pAC-2 ou pAC-14) ou des gènes précoces ou tardifs de l'adénovirus 2, etc. L'insertion du fragment codant pour la sorbine derrière l'ATG du gène viral cloné avec son promoteur permet d'en assurer l'expression dans la cellule animale (après transfection, micro-injection ou fusion cellules-protoplastes), ou in vitro, la transcription par un extrait de cellules animales (cellules HeLa), ce qui conduit à la synthèse de l'ARN messager correspondant.

Exemple 4 : Etude de l'activité biologique

a) Action de la sorbine et des peptides de synthèse C terminaux sur les mouvements d'eau et des électrolytes duodénaux

Chez le rat privé d'aliment depuis 45 heures mais recevant de l'eau à volonté, une anesthésie est assurée par le pentobarbital sodique, un cathéter est placé sur une jugulaire, trois anses duodénale, jéjunale et iléale sont constituées entre deux ligatures. Un ml de solution test contenant 70 mM de NaCl, 5,2 mM de KCl, 1,2 nM de $CaCl_2$, 10 mM de $CO_3Na$ ET 136 mM de mannitol est instillé dans chaque anse, le PEG 4000 marqué au $^3H$ est utilisé comme marqueur non absorbable, le $^{22}Na$ ou le $^{36}Cl$ comme marqueurs des flux bidirectionnels de sodium ou de chlore. La sorbine ou les peptides de synthèse sont administrés par voie veineuse dans du NaCl à 0,9%, à un débit de 3 ml/h. Après une heure de contact, le liquide restant dans la lumière de l'anse est collecté, le volume et les concentrations de Na, Ca, K, Cl et $HCO_3$ ainsi que la radioactivité liée au tritium, au chlore ou au sodium sont mesurés.

Les résultats obtenus sont rapportés dans le tableau suivant :

| | DOSE pmol/100g | Eau ml | Cl $\mu$eq | Na $\mu$eq |
|---|---|---|---|---|
| TEMOINS | 0 | 0 ±0,04 | 0 ±6,5 | 0 ±6,6 |
| HEPTAPEPTIDE | 50 | -0,20 ±0,07 | -36,1 ±10,3 | -30,7 ±10,0 |
| HEPTAPEPTIDE AMIDE | 50 | -0,19 ±0,04 | -37,2 ±3,8 | -32,6 ±5,4 |
| DECAPEPTIDE AMIDE | 12.5 | -0,34·· ±0,04 | -43,1·· ±5,2 | -48,5·· ±6,3 |
| ICOSAPEPTIDE AMIDE | 50 | -0,22·· ±0,05 | -32,7·· ±7,5 | -34,2·· ±7,1 |
| DECAPEPTIDE-GLYCOCOLLE | 50 50 | + 0,04 ±0,19 | + 8,3 ±34,8 | + 16,6 ±34,0 |
| SORBINE | 25 | -0,29· ±0,01 | -51,6· ±11,5 | -42,7 ±12,9 |
| ANGIOTENSINE II | 64 | -0,29· ±0,08 | -42,8· ±10,2 | -43,8 ±11,5 |
| METENKEPHALIN-AMIDE | 8000 | -0,31· ±0,05 | -43,0· ±6,7 | -49,7· ±6,7 |

L'absorption est indiquée par un signe (-), la sécrétion par un signe ( + ). les résultats concernent des groupes de 8 animaux ou plus M ± SEM · P<0.05 ·· P<0.01

L'examen des résultats donnés dans le tableau montre que la sorbine et les peptides de synthèse augmentent l'absorption des électrolytes et de l'eau dans le duodénum et le jejunum de rat. On constate que l'efficacité au niveau du duodénum des différents peptides obtenus est équivalente à celle de doses 4 fois supérieures d'angiotensine II, et 500 fois supérieures de métenképhalinamide, peptides de référence.

D'une manière avantageuse, la sorbine et ses peptides terminaux n'entraînent pas d'augmentation de la tension artérielle mesurée par cathétérisme de la carotide, à la différence de l'angiotensine II. La sorbine et ses peptides C terminaux n'entraînent pas d'inhibition de la contraction intestinale stimulée électriquement, à la différence de la métenképhalinamide. On constate que le décapeptide, possédant un acide aminé supplémentaire en position C-terminal, à savoir un motif glycocollé, en particulier, dont le radical amide est remplacé par le glycocolle possède un effet antagoniste de la sorbine et des peptides C terminaux en induisant une sécrétion au lieu d'une absorption.

B - Action sur la vésicule biliaire isolée

La vésicule biliaire de cobaye a été prélevée dès la mort des animaux par rupture cervicale. La vésicule a été remplie de solution de KREBS et placée dans un bain à base de solution de KREBS et alimenté en bulles d'un mélange oxygène 95%, gaz carbonique 5%. L'absorption a été déterminée par la courbe pondérale des vésicules pesées toutes les 5 minutes quand la sorbine est ajoutée à la solution de KREBS. Une accélération de la perte de poids par rapport à la période témoin représente une augmentation de l'absorption qui se traduit par une diminution du rapport perte de poids horaire témoin sur perte de poids horaire sous traitement.

REPARTITION DE LA SORBINE NATURELLE CHEZ L'HOMME ET LE PORC

La sorbine et les fragments de synthèse depuis de l'heptapeptide à l'icosapeptide ont été injectés à des lapins plusieurs fois jusqu'à l'obtention d'anticorps spécifiques. Un dosage radioimmunologique a pu être emis au point et a permis de mesurer dans les tissus la quantité de sorbine. Celle-ci est répartie assez largement en quantités réduites mais avec une prédominence notable au niveau de l'hypophyse, des surrénales, des bronches et du duodénum.

**Revendications**

1. Polypeptides et peptides dérivés capables notamment de provoquer un accroissement de l'absorption par les muqueuses, caractérisés en ce qu'ils comportent la séquence (I) suivante en acides aminés :

```
    1     5     10      15   20      25     30     35     40
>MRAATPLQTVDRPKDTYKTMFKQIHMVHKPDDDTKMYNTP
       45      50  55      60     65     70     75     80
YTYNAGLYNSPYSAQSHPAAKTQTYRPLSKSHSDNGTDAF
       85      90     95     100    105    110    115    120
KDASSPVPPPHVPPPVPPLRPRDRSSTEKHDRDPPDRKVD
      125    130    135    140    145    150 153
TRKFRSEPRSIFEYEPGKSSILQHERPVTKPQA- NH2
```

ou une partie de cette séquence sous réserve que les polypeptides ou peptides formés soient capables notamment de provoquer un accroissement de l'absorption par les muqueuses.

2. Polypeptides et peptides dérivés, caractérisés en ce qu'ils renferment au moins une partie d'un heptapeptide de séquence (II) suivante et en ce qu'ils sont capables notamment de provoquer un accroissement de l'absorption par les muqueuses

P.V.T.K.P.Q.A - NH$_2$

3. Polypeptides et peptides dérivés, caractérisés en ce qu'ils renferment au moins une partie d'un décapeptide répondant à la séquence (III) suivante en acides aminés et en ce qu'ils sont capables notamment de provoquer un accroissement de l'absorption par les muqueuses

H.E.R.P.V.T.K.P.Q.A - NH$_2$

4. Polypeptides et peptides dérivés, caractérisés en ce qu'ils renferment au moins une partie de la séquence (IV) de 40 acides aminés et en ce qu'ils sont capables notamment de provoquer un accroissement de l'absorption par les muqueuses

```
      1        5        10        15        20        25
      P.P.D.R.K.V.D.T.R.K.F.R.S.E.P.R.S.I.F.E.Y.E.P.G.K.S.S.I.
       30        35        40
      L.Q.H.E.R.P.V.T.K.P.Q.A - NH₂
```

5. Sorbine de pureté élevée, capable notamment de provoquer un accroissement de l'absorption par les muqueuses, répondant aux caractéristiques selon l'une quelconque des revendications 1 à 4, présentant un seul pic en HPLC avec un temps de rétention de 20 à 22 minutes sur une colonne micro-Bondapak® C18, Waters (3,9 x 300 mm), avec un débit de 1,5 ml/minute, en utilisant un solvant A constitué par un méthanol de 40% et de TFA à 0,1% et un solvant B formé de méthanol de 80% et de TFA à 0,1%, le gradient de B étant de 0 à 75% en 30 minutes.

6. Sorbine de pureté élevée capable notamment de provoquer un accroissement de l'absorption par les muqueuses, répondant aux caractéristiques selon l'une quelconque des revendications 1 à 4, présentant un seul pic en HPLC avec un temps de rétention de 15 à 16 minutes sur une colonne micro-Bondapak® C18, Waters (3,9 x 300 mm), avec un débit de 1,5 ml/minute, en utilisant un solvant A constitué par un méthanol de 40% et de TFA à 0,1% et un solvant B formé de méthanol de 80% et de TFA à 0,1%, le gradient de B étant de 0 à 100% en 20 minutes.

7. Sorbine selon la revendication 5 ou 6, caractérisée en ce qu'elle répond à la séquence (I) selon la revendication 1.

8. Décapeptide répondant à la séquence (III) selon la revendication 3.

9. Anticorps capables de reconnaître spécifiquement un polypeptide ou un peptide selon l'une quelconque des revendications 1 à 8.

10. Séquences d'ARN-m correspondant aux polypeptides et peptides dérivés selon l'une quelconque des revendications 1 à 8.

11. Fragments d'ADN capables de coder pour au moins une partie des polypeptides et de leurs dérivés selon l'une quelconque des revendications 1 à 8, caractérisés par leur capacité d'hybridation avec un gène capable d'exprimer au moins une partie de la sorbine de séquence (I).

12. Procédé d'obtention de polypeptides selon l'une quelconque des revendications 1 à 8, par extraction, à partir d'organes de mammifères, de peptides à l'aide d'un acide tel que l'acide acétique, relargage par un sel tel que le chlorure de sodium, puis purification par précipitation alcoolique, caractérisé en ce que le précipité est soumis aux étapes suivantes :
   - purification par chromatographie sur carboxyméthylcellulose (CMC) avec élution par du bicarbonate d'ammonium 0,04M,
   - récupération de la fraction active et nouvelle chromatographie sur CMC, avec élution par du bicarbonate d'ammonium 0,03M,
   - récupération de la fraction active soumise à au moins à une purification par HPLC avec un solvant A constitué par de l'eau avec 0,1% d'acide trifluoroacétique et un solvant B constitué par de l'acétonitrile contenant 0,1% de TFA.

13. Procédé selon la revendication 12, caractérisé, en ce que les purifications successives par HPLC sont réalisées comme suit :
- on utilise une colonne micro-Bondapak® C 18 waters (7,8 x 300 mm) et un gradient du solvant B de 0 à 20% en 10 minutes puis de 20 à 40 en 40 minutes et un débit de 3 ml/minute, on élue le produit actif avec un temps de rétention de 37 à 39 minutes,
- on utilise une colonne micro-Bondapak® CN Waters (3,9 x 300 mm), avec un débit de 1,5 ml/minute et un solvant A constitué par de l'eau et 0,1% de TFA et un solvant B comprenant de l'acétonitrile et 0,1% de TFA, on ajoute le solvant B en gradient à raison de 0 à 20% en 10 minutes, puis 20 à 40% en 40 minutes, on élue la fraction active avec un temps de rétention de 37 à 41 minutes,
- on utilise une colonne micro-Bondapak® C18, Waters (3,9 x 300 mm) avec un débit de 1,5 ml/minute, un solvant A, formé par du méthanol 40% et du TFA 0,1%, un solvant B constitué par du méthanol 80% et du TFA 0,1%, le gradient de B étant de 0 à 75% en 30 minutes, le temps de rétention de la sorbine étant de 20 à 22 minutes.

14. Compositions pharmaceutiques, caractérisées en ce qu'elles renferment une quantité efficace d'au moins un polypeptide ou peptide dérivé selon l'une quelconque des revendications 1 à 8.

**Claims**

1. Polypeptide and peptide derivatives capable in particular of bringing about an increase in absorption by the mucosae characterized in that they comprise the following amino acid sequence (1) :

```
 1     5    10    15  20   25   30   35   40
>MRAATPLQTVDRPKDTYKTMFKQIHMVHKPDDDTKMYNTP
    45    50  55   60   65   70   75   80
YTYNAGLYNSPYSAQSHPAAKTQTYRPLSKSHSDNGTDAF
    85    90   95   100  105  110  115  120
KDASSPVPPPHVPPPVPPLRPRDRSSTEKHDRDPPDRKVD
   125   130  135  140  145  150 153
TRKFRSEPRSIFEYEPGKSSILQHERPVTKPQA- NH2
```

or a part thereof providing that the polypeptides or peptides formed are capable especially of bringing about an increase in absorption by the mucosae.

2. Polypeptide and peptide derivatives, characterized in that they contain at least a part of a heptapeptide of the following sequence (II) and in that they are capable of bringing about an increase in absorption by the mucosae.
P.V.T.K.P.Q.A. - NH$_2$

3. Polypeptides and peptide derivatives characterized in that they contain at least one part of the decapeptide corresponding to the following amino acid sequence (III) and in that they are capable of bringing about an increase in absorption by the mucosae.
H.E.R.P.V.T.K.P.Q.A. - NH$_2$

4. Polypeptides and peptide derivatives characterized in that they contain at least a part of the sequence (IV) of 40 amino acids and in that they are capable of bringing about an increase in absorption by the mucosae.

13

```
      1         5          10          15          20          25
P.P.D.R.K.V.D.T.R.K.F.R.S.E.P.R.S.I.F.E.Y.E.P.G.K.S.S.I.
      30          35          40
L.Q.H.E.R.P.V.T.K.P.Q.A-NH₂
```

5. Sorbin of high purity capable, in particular of bringing about an increase in absorption by the mucosae, possessing the characteristics according to anyone of claims 1 to 4, showing a single peak in HPLC with a retention time of 20 to 22 minutes on a Waters micro-Bondapak C18 column (3.9 x 300 mm), with a flow rate of 1.5 ml/minute, using a solvent A consisting of 40% methanol and 0.1% of TFA and a solvent B composed of 80% methanol and 0.1% of TFA, the gradient of B passing from 0 to 75% in 30 minutes.

6. Sorbin of high purity capable, in particular of bringing about an increase in absorption by the mucosae, possessing the characteristics according to anyone of claims 1 to 4, showing a single peak in HPLC with a retention time of 15 to 16 minutes on a Waters micro-Bondapak C18 column (3.9 x 300 mm), with a flow rate of 1.5 ml/minute, using a solvent A consisting of 40% methanol and 0.1% of TFA and a solvent B composed of 80% methanol and 0.1% of TFA, the gradient of B passing from 0 to 100% in 20 minutes.

7. Sorbin according to claims 5 or 6 characterized in that it corresponds to the sequence (I) according to claim 1.

8. Decapeptide corresponding to the sequence (II) according to claim 3.

9. Antibodies capable of specifically recognizing a polypeptide or a peptide according to anyone of claims 1 to 8.

10. mRNA sequences corresponding to the polypeptides and peptide derivatives according to anyone of claim 1 to 8.

11. DNA fragments capable of encoding at least a part of the polypeptides and their derivatives according to anyone of claims 1 to 8, characterized by their capacity to hybridize with a gene capable of expressing at least a part of the sorbin sequence (I).

12. Procedure for the preparation of polypeptides according to anyone of claims 1 to 8, by extraction from mammalian organs of peptides with the aid of an acid such as acetic acid, salting out by a salt such as sodium chloride, then purification by alcoholic precipitation, characterized in that the precipitate is subjected to the following steps :
    - purification by chromatography of carboxymethylcellulose (CMC) with elution by 0.04M ammonium bicarbonate,
    - recovery of the active fraction and repetition of the chromatography on CMC, with elution by 0.03M ammonium bicarbonate,
    - recovery of the active fraction subjected to at least one purification by HPLC with a solvent A constituted by water with 0.1% of trifluoroacetic acid and a solvent B constituted by acetronile containing 0.1% of TFA.

13. Procedure according to claim 12, characterized in that the successive purifications by HPLC are carried out as follows :
    - a Waters micro-Bondapak C18 column (7.8 x 300 mm) is used with a gradient of solvent B of 0 to 20% in 10 minutes followed by 20 to 40% in 40 minutes, and a flow rate of 3 ml/minute, and the active product is eluted with a retention time of 37 to 39 minutes,
    - a Waters micro-Bondapak C18 column (3.9 x 300 mm) is used with a flow rate of 1.5 ml/minute and a solvent A constituted by water and 0.1% of TFA and a solvent B is introduced as a gradient of 0 to 20% in 10 minutes, then 20 to 40% in 40 minutes, and the active fraction is eluted with a retention time of 37 to 41 minutes,
- a Waters micro-Bondapak C18 column (7.8 x 300 mm) is used with a flow rate of 1.5 ml/minute, a solvent A, consisting of 40% methanol and 0.1% of TFA, a solvent B consisting of 80% methanol and 0.1% of TFA, the gradient B passing from 0 to 75% in 30 minutes, and the retention time of sorbin being 20 to 22 minutes.

14. Pharmaceutical compositions, characterized in that they contain an efficacious amount of at least one polypeptide or peptide derivative according to anyone of claims 1 to 8.

**Patentansprüche**

1. Polypeptide und Peptidderivate, die insbesondere zur Erhöhung der Absorptionsfähigkeit der Schleimhäute befähigt sind, dadurch gekennzeichnet, daß sie die nachstehende Aminosäuresequenz (I)

```
    1      5     10    15  20   25   30   35   40
>MRAATPLQTVDRPKDTYKTMFKQIHMVHKPDDDTKMYNTP
      45      50  55    60    65   70    75   80
YTYNAGLYNSPYSAQSHPAAKTQTYRPLSKSHSDNGTDAF
      85    90    95   100   105  110   115  120
KDASSPVPPPHVPPPVPPLRPRDRSSTEKHDRDPPDRKVD
     125   130   135   140   145   150  153
TRKFRSEPRSIFEYEPGKSSILQHERPVTKPQA- NH2
```

oder einen Teil dieser Sequenz umfassen, mit der Maßgabe, daß die erhaltenen Polypeptide oder Peptide insbesondere zur Erhöhung der Absorptionsfähigkeit der Schleimhäute befähigt sind.

2. Polypeptide und Peptidderivate, dadurch gekennzeichnet, daß sie mindestens einen Teil des Heptapeptids der nachstehenden Sequenz (II) umfassen und insbesondere zur Erhöhung der Absorptionsfähigkeit der Schleimhäute befähigt sind:
P.V.T.K.P.Q.A - NH$_2$

3. Polypeptide und Peptidderivate, dadurch gekennzeichnet, daß sie mindestens einen Teil des Dekapeptids der nachstehenden Aminosäuresequenz (III) umfassen und insbesondere zur Erhöhung der Absorptionsfähigkeit der Schleimhäute befähigt sind:
H.E.R.P.V.T.K.P.Q.A - NH$_2$

4. Polypeptide und Peptidderivate, dadurch gekennzeichnet, daß sie mindestens einen Teil der aus 40 Aminosäuren bestehenden Sequenz (IV) umfassen und zur Erhöhung der Absorptionsfähigkeit der Schleimhäute befähigt sind:

```
  1      5       10        15       20       25
P.P.D.R.K.V.D.T.R.K.F.R.S.E.P.R.S.I.F.E.Y.E.P.G.K.S.S.I.
  30        35       40
L.Q.H.E.R.P.V.T.K.P.Q.A - NH2
```

5. Sorbine mit hohem Reinheitsgrad nach einem der Ansprüche 1 bis 4, das insbesondere zur Erhöhung der Absorptionsfähigkeit der Schleimhäute befähigt ist, und das bei der HPLC mit einer micro-Bondapak® C18-Säule, Waters (3,9 x 300 mm), und einem Durchfluß von 1,5 ml/Minute unter Verwendung eines aus 40%igem Methanol und 0,1% TFA bestehenden Lösungsmittels A und eines aus 80%igem Methanol und 0,1% TFA bestehenden Lösungsmittels B, nur ein Signal mit einer Retentionszeit von 20 bis 22 Minuten aufweist, wobei der Gradient von B innerhalb von 30 Minuten von 0 bis 75% erhöht wird.

6. Sorbine mit hohem Reinheitsgrad nach einem der Ansprüche 1 bis 4, das insbesondere zur Erhöhung der Absorptionsfähigkeit der Schleimhäute befähigt ist, das bei der HPLC mit einer micro-Bondapak® C18-Säule, Waters (3,9 x 300 mm), und einem Durchfluß von 1,5 ml/Minute unter Verwendung eines aus 40%igem Methanol und 0,1% TFA bestehenden Lösungsmittels A und eines aus 80%igem Methanol und 0,1% TFA bestehenden Lösungsmittels B nur ein Signal mit einer Retentionszeit von 15 bis 16 Minuten aufweist, wobei der Gradient von B innerhalb von 20 Minuten von 0 bis 100% erhöht wird.

7. Sorbine nach Anspruch 5 oder 6, dadurch gekennzeichnet, daß es der Sequenz (I) von Anspruch 1 entspricht.

8. Dekapeptid, entsprechend der Sequenz (III) von Anspruch 3.

9. Antikörper, der zur Erkennung eines spezifischen Polypeptids oder Peptids nach einem der Ansprüche 1 bis 8 befähigt ist.

10. mRNA-Sequenzen, die den Polypeptiden und Peptidderivaten nach einem der Ansprüche 1 bis 8 entsprechen.

11. DNA-Fragmente, die mindestens einen Teil der Polypeptide und ihrer Derivate nach einem der Ansprüche 1 bis 8 kodieren, gekennzeichnet durch ihre Fähigkeit zur Hybridisierung mit einem Gen, welches zur Expression von mindestens einem Teil des Sorbines der Sequenz (I) befähigt ist.

12. Verfahren zur Herstellung von Polypeptiden nach einem der Ansprüche 1 bis 8, wobei mit Hilfe einer Säure wie Essigsäure Peptide aus Säugetierorganen extrahiert werden, diese mit Hilfe eines Salzes wie Natriumchlorid ausgesalzt und durch Ausfällen mittels eines Alkohols gereinigt werden, dadurch gekennzeichnet, daß der Niederschlag folgenden Schritten unterworfen wird:
- Reinigung durch Chromatographie über Carboxymethylcellulose (CMC) mit Elution durch Ammoniumbicarbonat 0,04M,
Rückgewinnung der aktiven Fraktion und erneute Chromatographie über CMC mit Elution durch Ammoniumbicarbonat 0,03M,
- Rückgewinnung der aktiven Fraktion, die mindestens einem Reinigungsschritt durch HPLC unter Verwendung eines aus Wasser und 0,1% Trifluoressigsäure bestehenden Lösungsmittels A und eines aus Acetonitril und 0,1% TFA bestehenden Lösungsmittels B unterworfen wird.

13. Verfahren nach Anspruch 12, dadurch gekennzeichnet, daß die nachstehenden, aufeinanderfolgenden Reinigungsschritte mit HPLC durchgeführt werden:
- Verwendung einer micro-Bondapak® C18-Säule, Waters (7,8 x 300 mm), und eines Gradienten für das Lösungsmittel B von 0 bis 20% innerhalb von 10 Minuten und von 20 bis 40% innerhalb von 40 Minuten bei einem Durchfluß von 3 ml/Minute, wobei die aktive Substanz mit einer Retentionszeit von 37 bis 39 Minuten eluiert wird,
- Verwendung einer micro-Bondapak® CN-Säule, Waters (3,9 x 300 mm), bei einem Durchfluß von 1,5 ml/Minute, mit einem aus Wasser und 0,1% TFA bestehenden Lösungsmittel A und einem aus Acetonitril und 0,1% TFA bestehenden Lösungsmittel B, wobei das Lösungsmittel B mit einem Gradienten von 0 bis 20% innerhalb von 10 Minuten und von 20 bis 40% innerhalb von 40 Minuten zugegeben wird und die aktive Fraktion mit einer Retentionszeit von 37 bis 41 Minuten eluiert wird,
- Verwendung einer micro-Bondapak® C18-Säule, Waters 3,9 x 300 mm), bei einem Durchfluß von 1,5 ml/Minute, mit einem aus 40%igem Methanol und 0,1% TFA bestehenden Lösungsmittel A und einem aus 80%igem Methanol und 0,1% TFA bestehenden Lösungsmittel B, wobei der Gradient von B innerhalb von 30 Minuten von 0 bis 75% erhöht wird und die Retentionszeit des Sorbines 20 bis 22 Minuten beträgt.

**14.** Arzneimittel, dadurch gekennzeichnet, daß es eine wirksame Menge von mindestens einem Polypeptid oder Peptidderivat nach einem der Ansprüche 1 bis 8 umfaßt.

17